# EUROPEAN PATENT APPLICATION

(11) **EP 4 434 570 A1**
(43) Date of publication of application: **25.09.2024**
(21) Application number: 22909862.9
(22) Date of filing: 15.12.2022
(51) Int. Cl.: A61M 37/00

(54) **MICRONEEDLE ARRAY CHIP**

(30) Priority: 23.12.2021 CN 202123259085 U
(71) Applicant: Suzhou Nasheng Microelectronics Co., Ltd, Suzhou, Jiangsu 215000 (CN)
(72) Inventor: XU, Bai, Suzhou, Jiangsu 215000 (CN)
(74) Representative: Perronace, Andrea
(86) International application number: PCT/CN2022/139300
(87) International publication number: WO 2023/116548

(57) **Abstract**

Disclosed in the present invention is a microneedle array chip, comprising a substrate and a plurality of microneedles arranged on a front side of the substrate in an array, wherein a notch is formed downwards from a tip of each microneedle; the width of the notch is greater than or equal to 10 nm; and the depth thereof is greater than or equal to 50 nm. A microneedle array chip, comprising a substrate, wherein a plurality of microneedle clusters are arranged on a front side of the substrate in an array; each microneedle cluster is composed of at least two microneedles; and a gap between the microneedles in each microneedle cluster is greater than or equal to 10 nm, but less than or equal to 300 µm. By means of the notches in the microneedles or the gaps between the microneedles in the microneedle clusters, the microneedles can play a role in carrying a medicament or a beauty product, and after the microneedles pass through the stratum corneum, the medicament or the beauty product can be carried into the deep tissue below the stratum corneum of the skin, thereby improving the absorption efficiency of the medicament or the beauty product.

## Description

### TECHNICAL FIELD

The present application relates to the field of transdermal administration technologies, and specifically relates to a microneedle array chip.

### BACKGROUND ART

The microneedle array chip, a device that uses microneedles to pass through the stratum corneum of the skin, can be used in the medical or cosmetic field, and can make a medicament or a beauty product pass through the stratum corneum of the skin, so as to achieve a better absorption effect. In this technical field, a great deal of prior art records technical information on the microneedles, such as preparation, material, structure, use and development, for example, CN100355470C, CN100402107C, CN103079634B, CN100408126C, CN100528091C, CN109432585A, CN111300702A, CN105581975A and many other patent documents.

Microneedles in the conventional microneedle array chips are divided into two types, solid microneedles and hollow microneedles respectively.

The solid microneedle may produce micro-channels in the stratum corneum through reciprocating piercing. The channels may last for a short period of time, during which period, the medicament or beauty product is applied to the skin, and then the medicament or the beauty product may pass through the stratum corneum of the skin through the micro-channels, so as to be absorbed by deep tissue of the skin. However, the absorption efficiency of such mode is low.

The working principle of the hollow microneedle is similar to that of a syringe. After piercing the stratum corneum of the skin, the hollow microneedle stays in the stratum corneum of the skin, the medicament is injected into the deep tissue of the skin by an injection system through a cavity of the hollow microneedle. However, the hollow microneedle is complex in fabrication process, low in yield, and high in production cost.

A soluble microneedle is made of an organic material, and the medicament or the beauty product is solidified on a surface of the microneedle. The soluble microneedle, after passing through the stratum corneum, stays in the stratum corneum of the skin for several minutes to several hours, within which period, the medicament or the beauty product is continuously dissolved in the deep tissue of the skin. However, the soluble microneedle is complex in fabrication process and high in cost.

Therefore, the prior art in the field of microneedle transdermal administration technologies requires improvement.

### SUMMARY

### TECHNICAL PROBLEM

The technical problem to be solved by the present application is to provide a solid microneedle array chip which can improve the efficiency of a medicament or a beauty product passing through the stratum corneum of the skin.

### SOLUTION TO PROBLEM

### TECHNICAL SOLUTION

In order to solve the above technical problem, technical solutions adopted in the present application are as follows.

A microneedle array chip, including a substrate and a plurality of microneedles arranged on a front side of the substrate in an array, wherein trenches are formed downwards from tips of the microneedles, and wherein each of the trenches has a width not less than 10 nm, and a depth not less than 50 nm.

Definition: the microneedle is a micro needle with a needle length of less than 5 mm, a material of the microneedle may be monocrystalline silicon, metal or organic material, and a size of the microneedle may be on the order of micron or nanometer.

Preferably, a back side of the substrate is provided with medicine storage reservoirs, and the medicine storage reservoirs and the trenches realize fluid communication through through holes.

Preferably, the microneedle array chip further includes through holes extending from bottom surfaces of the trenches to the back side of the substrate.

Preferably, the trenches are each a straight trench or formed by two straight trenches intersecting by any angle.

A microneedle array chip, including a substrate, wherein a front side of the substrate is provided with a plurality of microneedle clusters arranged in an array, and wherein each of the microneedle clusters is composed of at least two microneedles, a gap between the microneedles in each microneedle cluster is not less than 10 nm and not greater than 300 µm.

Definition: the microneedle cluster refers to a group of microneedles, wherein the number of microneedles in one microneedle cluster is not less than 2. A gap between the microneedles in the microneedle cluster is less than an interval between the microneedle clusters on the microneedle array chip.

Preferably, a back side of the substrate is provided with medicine storage reservoirs, bottom surfaces of the medicine storage reservoirs and the front side of the substrate realize fluid connection through through holes, and the through holes are located in positions between the microneedles in the microneedle clusters.

Preferably, each of the microneedle clusters is composed of two microneedles.

Preferably, each of the microneedle clusters is composed of more than two microneedles, and the microneedles are circumferentially arranged.

### BENEFICIAL EFFECTS OF PRESENT APPLICATION

### BENEFICIAL EFFECTS

The beneficial effects of the present application are as follows: through the trenches on the microneedles or the gaps between the microneedles in the microneedle clusters, the microneedles may have a function of carrying a medicament or a beauty product, and when passing through the stratum corneum, the microneedles can carry the medicament or the beauty product into deep tissues below the stratum corneum of the skin, so as to improve the absorption efficiency of the medicament or the beauty product.

### BRIEF DESCRIPTION OF DRAWINGS

### DRAWING DESCRIPTION

FIG. 1 is a front schematic diagram of a microneedle chip array of Embodiment 1;
FIG. 2 is a back schematic diagram of a microneedle chip array of Embodiment 1;
FIG. 3 is a partial schematic diagram of a single microneedle cluster in Embodiment 1;
FIG. 4 is a partial schematic diagram of a single medicine storage reservoir in Embodiment 1;
FIG. 5 is a sectional view of a single microneedle cluster in Embodiment 1;
FIG. 6 is a partial schematic diagram of a single microneedle cluster in Embodiment 2;
FIG. 7 is a partial schematic diagram of a single microneedle cluster in Embodiment 3;
FIG. 8 is a first partial lateral schematic diagram of a single microneedle cluster in Embodiment 3;
FIG. 9 is a second partial lateral schematic diagram of a single microneedle cluster in Embodiment 3;
FIG. 10 is a partial schematic diagram of a single microneedle cluster in Embodiment 4;
FIG. 11 is a partial lateral schematic diagram of a single microneedle cluster in Embodiment 4;
FIG. 12 is a partial schematic diagram of a single microneedle cluster in Embodiment 5; and
FIG. 13 is a sectional view of a single microneedle cluster in Embodiment 5.

### Reference sign list:

1. substrate; 2. microneedle cluster; 21. microneedle; 22. tip portion; 23. trench; 3. medicine storage reservoir; 4. shallow groove; 5. through hole.

### OPTIMAL EMBODIMENTS FOR IMPLEMENTING PRESENT APPLICATION

### OPTIMAL EMBOMENTS OF PRESENT APPLICATION

The technical solutions in the embodiments of the present application will be clearly and completely described below in conjunction with the drawings in the embodiments of the present application. Apparently, some but not all embodiments of the present application are described.

### Embodiment 1 of Microneedle Array Chip

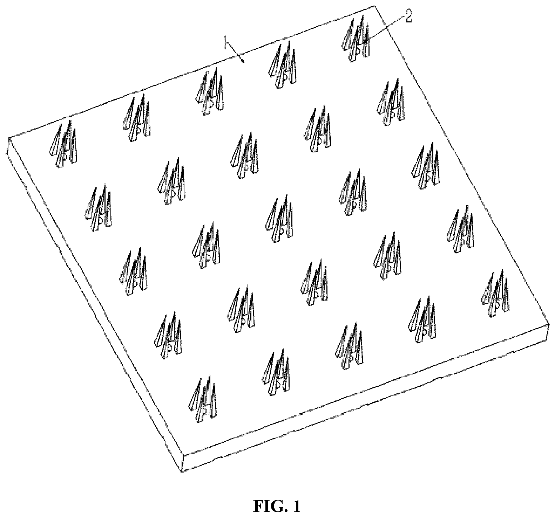

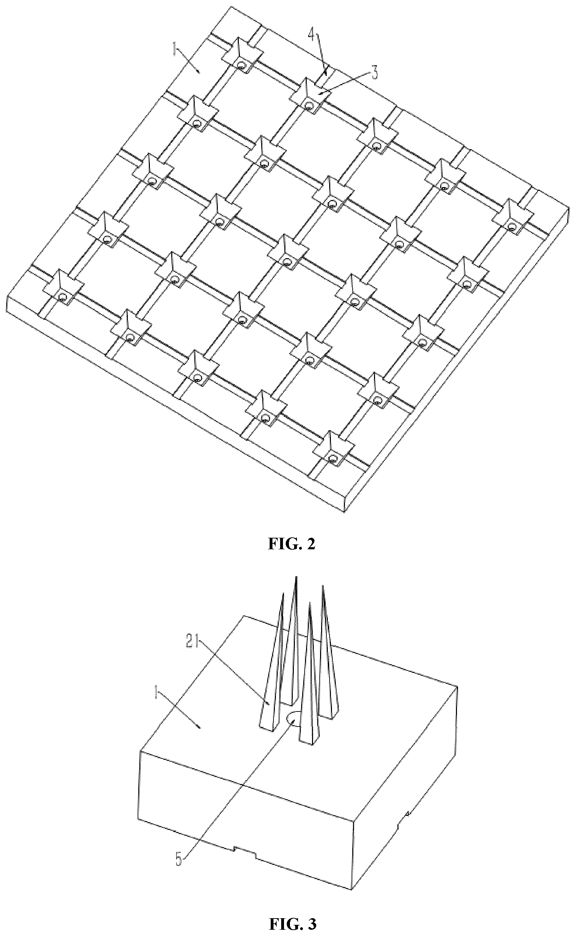

A microneedle array chip is as shown in FIG. 1, wherein a front side of a substrate 1 is provided with a plurality of microneedle clusters 2, the microneedle clusters 2 are arranged in a rectangular array, and both horizontal and vertical intervals between the plurality of microneedle clusters 2 are 400 microns. The microneedle clusters 2 each are of a structure as shown in FIG. 3, wherein in the microneedle cluster 2, four microneedles 21 are gathered together, and are arranged in a circumferential array or are centrosymmetrically arranged, and the microneedles 21 form cross-shaped channels therebetween, and wherein the channels also may be considered as being formed through intersection of two rectangular channels perpendicular to each other. In the microneedle cluster 2, each microneedle 21 is a rectangular pyramid, and has a height of 300 microns, and a root cross section of the microneedle 21 is in a square shape with a side length of 25 microns. A gap between each two adjacent microneedles 21 is 50 microns. It may also be understood as that the cross-shaped channels are each formed by two 50-micron wide rectangular channels perpendicular to each other. A back side of the substrate 1 is provided with medicine storage reservoirs 3 (the medicine storage reservoirs being recessed inwards). The medicine storage reservoirs 3 are each connected to the front side of the substrate through a liquid guiding hole (also called a through hole). The liquid guiding holes make the front side of the substrate in fluid communication with the medicine storage reservoirs. Each liquid guiding hole has a diameter of 40 microns, and the liquid guiding hole is located in a gap between the microneedles 21 in the microneedle cluster 2, i.e., in the cross-shaped channels.

Compared with the existing solid microneedle array chip, in the microneedle array chip provided in the present embodiment, the microneedle clusters 2 are used to replace the solid microneedles in the prior art. The microneedle clusters 2 pass through the skin by the same function principle as that of the solid microneedles, and are used to pass through the stratum corneum of the skin, preferably, pass through the stratum corneum by generating high-frequency vibration by permeation-promoting instrument or other devices to make micropores left in the stratum corneum, and then an active ingredient for cosmetic use or a medicinal ingredient for therapeutic use can pass through the stratum corneum of the skin through the micropores, and enter deep structures of the skin, so as to improve the absorption effect of the active ingredient for cosmetic use or the medicinal ingredient for therapeutic use. Compared with the prior art, the present embodiment further achieves the following technical progress: the gaps between the microneedles 21 in the microneedle clusters 2 may achieve a certain carrying effect, and in a process of the microneedle clusters 2 reciprocatingly piercing the skin, the gaps may carry the active ingredient for cosmetic use or the medicinal ingredient for therapeutic use into the deep structures of the skin, while the solid microneedles in the prior art do not have such carrying capability. Therefore, compared with the solid microneedle array chips in the prior art, the present embodiment may further improve the absorption effect of the active ingredient for cosmetic use or the medicinal ingredient for therapeutic use.

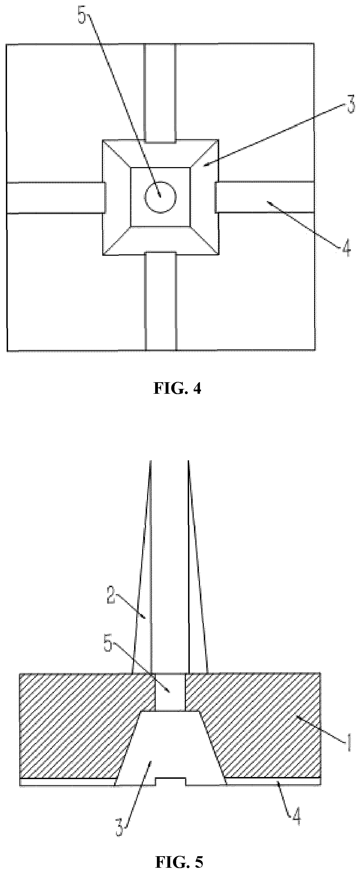

Compared with the existing solid microneedle array chips, in the present embodiment, the back side of the substrate 1 is further additionally provided with the medicine storage reservoirs 3. As shown in FIGS. 2, 4 and 5, the medicine storage reservoirs 3 may store a certain amount of the active ingredient for cosmetic use or the medicinal ingredient for therapeutic use. The active ingredient for cosmetic use or the medicinal ingredient for therapeutic use reaches the front side of the substrate 1 and between the channels of the microneedle clusters 2 through the liquid guiding holes, and passes through the stratum corneum along with the microneedle clusters 2 in the process of piercing the stratum corneum, and enters the deep structures of the skin. In this way, a liquid-instrument integrated design is realized. Before using the microneedle array chip, it is unnecessary to apply the active ingredient for cosmetic use or the medicinal ingredient for therapeutic use in advance on a skin surface, thereby achieving the effects of simplifying operation steps and reducing users' learning costs. The mechanism by which the active ingredient for cosmetic use or the medicinal ingredient for therapeutic use passes through the liquid guiding holes is similar to the mechanism by which a liquid in a device such as liquid-instrument integrated permeation-promoting instrument or a vital injector is discharged from a liquid outlet head, wherein during reciprocating vibration of the microneedle array chip, the active ingredient for cosmetic use or the medicinal ingredient for therapeutic use is thrown out from the liquid guiding holes due to its own inertia. Each microneedle cluster 2 is provided with one corresponding medicine storage reservoir 3, and the medicine storage reservoirs 3 are connected by shallow grooves 4, so that air pressures in the medicine storage reservoirs 3 are the same, and liquid discharge effects of the microneedle clusters 2 are consistent.

### EMBODIMENTS

### EMBODIMENTS OF PRESENT APPLICATION

### Embodiment 2 of Microneedle Array Chip

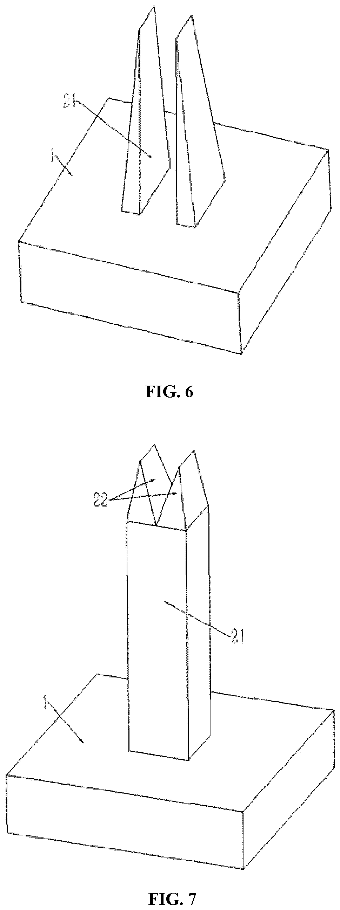

A front side of a substrate 1 is provided with a plurality of microneedle clusters 2, wherein the microneedle clusters 2 are arranged in a rectangular array, and both horizontal and vertical intervals between the microneedle clusters 2 are 300 microns. Each microneedle cluster 2 is of a structure as shown in FIG. 6, wherein the microneedle cluster 2 is composed of two microneedles 21, and the two microneedles are both blade-shaped. A height of the two microneedles is 300 microns, and a gap between the two microneedles is 50 microns.

Compared with Embodiment 1, in Embodiment 2, one microneedle cluster 2 is composed of two microneedles 21, and each microneedle 21 is blade-shaped, and also may be referred to as a micro blade. A back side of the substrate 1 is not provided with medicine storage reservoirs, and before using the microneedle array chip, an active ingredient for cosmetic use or a medicinal ingredient for therapeutic use needs to be applied in advance on a skin surface. In Embodiment 2, a gap between the two microneedles 21 also may have a certain carrying effect, and compared with the solid microneedle array chips in the prior art, an absorption effect of the active ingredient for cosmetic use or the medicinal ingredient for therapeutic use may be further improved.

### Embodiment 3 of Microneedle Array Chip

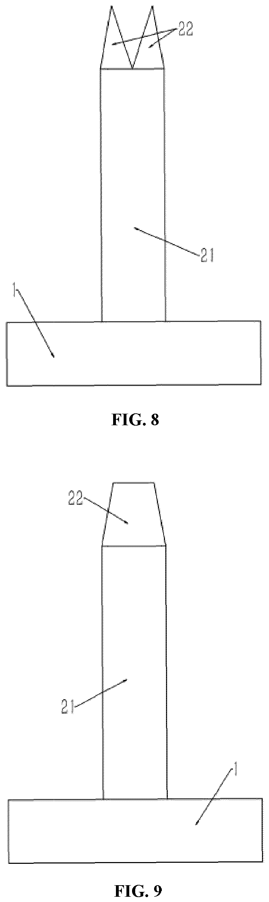

A front side of a substrate 1 is provided with a plurality of microneedles 21, wherein the microneedles 21 are arranged in a rectangular array, and both horizontal and vertical gaps between the microneedles 21 are 300 microns. Each microneedle 21 is of a structure as shown in FIGS. 7, 8 and 9, wherein a lower portion of a needle body of the microneedle is a prism, an upper portion thereof is formed to have two triangular tip portions 22, and a V-shaped trench 23 is formed between the two tip portions 22, wherein the trench has a depth of 100 µm, and the microneedle has a height of 400 µm. As shown in FIGS. 7, 8 and 9, the depth of the trench 23 is less than the height of the microneedle 21.

Compared with the solid microneedle arrays in the prior art, in the present embodiment, trench structures are formed in the tip portions 22 of the microneedles. The microneedles 21 pass through the skin by the same function principle as that of the solid microneedles, and are used to pass through the stratum corneum of the skin, preferably, pass through the stratum corneum by generating high-frequency vibration by permeation-promoting instrument or other devices to make micropores left in the stratum corneum, and then an active ingredient for cosmetic use or a medicinal ingredient for therapeutic use can pass through the stratum corneum of the skin through the micropores, and enter deep structures of the skin, so as to improve the absorption effect of the active ingredient for cosmetic use or the medicinal ingredient for therapeutic use. Compared with the prior art, the present embodiment further achieves the following technical progress: the trenches 23 in the tip portions 22 of the microneedles may achieve a certain carrying effect, and in a process of the microneedle reciprocatingly piercing the skin, the trenches 23 may carry the active ingredient for cosmetic use or the medicinal ingredient for therapeutic use into the deep structures of the skin, while the solid microneedles in the prior art do not have such carrying capability. Therefore, compared with the solid microneedle array chips in the prior art, the present embodiment may further improve the absorption effect of the active ingredient for cosmetic use or the medicinal ingredient for therapeutic use. Compared with Embodiment 1 or 2, the structure of forming the trenches in the tip portions 22 of the microneedles adopted in the present embodiment has less change to the structure of the existing solid microneedles and less change to the process, and is easy to apply. The trench structures less damage physical properties of the needle body of the microneedles, and may retain advantages of existing solid microneedle fabrication processes and advantages of manufactured solid microneedles, for example, the microneedles are not easy to break. It is easy to understand that when the depths of the trenches 23 formed downwards from the tip portions 22 of the microneedles are equal to the heights of the microneedles, the microneedles can form a structure of microneedle clusters, without increasing the difficulty of fabrication process.

### Embodiment 4 of Microneedle Array Chip

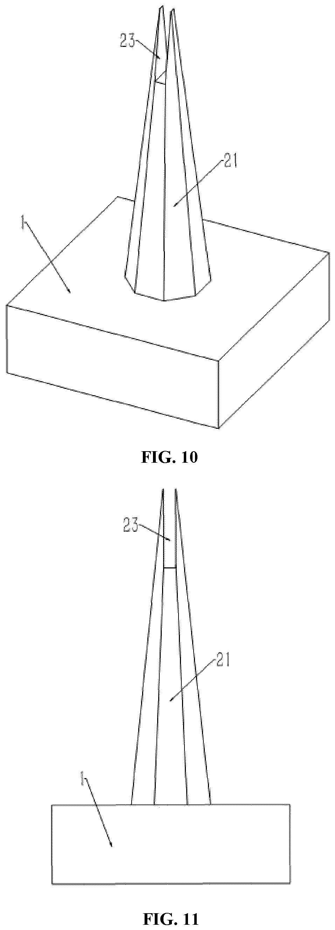

A front side of a substrate 1 is provided with a plurality of microneedles 21, wherein the microneedles 21 are arranged in a rectangular array, and both horizontal and vertical gaps between the microneedles 21 are 400 microns. Each microneedle is of a structure as shown in FIGS. 10 and 11, wherein the front side of the substrate 1 is provided with the microneedles 21 each having an outer contour of an octagonal truncated pyramid, and a top portion of each microneedle 21 is provided with a rectangular trench 23 extending from top to bottom, wherein a width of the trench 23 is 15 µm, a depth of the trench 23 is 100 µm, a height of the microneedle is 300 µm, a bottom diameter of the microneedle is 100 µm, and in this case, the depth of the trench 23 is less than the height of the microneedle. It is easy to understand that when the depths of the trenches 23 are equal to the heights of the microneedles 21, the microneedles in the present Embodiment 4 form a microneedle cluster structure similar to that in Embodiment 2. That is, the microneedle clusters 2 in Embodiment 2 may be understood as a special case of the present Embodiment 4. Likewise, the microneedle clusters in Embodiment 1 may be understood as being obtained by forming two intersecting straight trenches extending downwards from the tips of the microneedles until a height thereof is equal to that of the microneedles.

### Embodiment 5 of Microneedle Array Chip

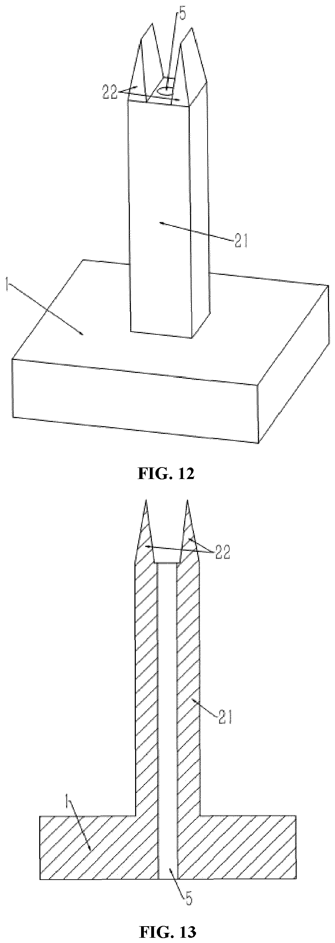

A front side of a substrate 1 is provided with a plurality of microneedles 21, wherein the microneedles 21 are arranged in a rectangular array, and both horizontal and vertical gaps between the microneedles 21 are 300 microns. Each microneedle is of a structure as shown in FIGS. 12 and 13, wherein a lower portion of a needle body of the microneedle 21 is a prism, an upper portion thereof is formed to have two triangular tip portions 22, and a trench 23 is formed between the two tip portions, wherein two side surfaces of the trench 23 are bevels, a bottom of the trench 23 is 40 microns wide, the trench 23 is 100 microns deep, a height of the microneedle is 400 microns, and the depth of the trench 23 is less than the height of the microneedle 21. A through hole 5 is formed from a back side of the substrate 1 to a bottom surface of the trench 23, so that the microneedle 21 becomes a hollow microneedle.

### Embodiment 6 of Microneedle Array Chip

A front side of a substrate is provided with a plurality of microneedle clusters 2, wherein the microneedle clusters 2 are arranged in a rectangular array, and both horizontal and vertical intervals between the microneedle clusters 2 are 300 microns. Each microneedle cluster 2 is composed of two microneedles 21, and the two microneedles 21 are both blade-shaped. The two microneedles 21 have a height of 300 microns, and a gap between the two microneedles is 10 nanometers.

### Embodiment 7 of Microneedle Array Chip

A front side of a substrate is provided with a plurality of microneedle clusters 2, wherein the microneedle clusters 2 are arranged in a rectangular array, and both horizontal and vertical intervals between the microneedle clusters 2 are 1000 microns. Each microneedle cluster 2 is composed of two microneedles 21, and the two microneedles 21 are both blade-shaped. The two microneedles 21 have a height of 500 microns, and a gap between the two microneedles 21 is 300 microns.

## Claims

1. A microneedle array chip, comprising a substrate and a plurality of microneedles arranged on a front side of the substrate in an array, **characterized in that** trenches are formed downwards from tips of the microneedles, and each of the trenches has a width not less than 10 nm, and a depth not less than 50 nm.

2. The microneedle array chip according to claim 1, wherein the microneedles each comprise an upper portion and a lower portion, wherein the upper portion is formed with tip portions, and the trench is formed between the tip portions.

3. The microneedle array chip according to claim 2, wherein the tip portions are triangular tip portions.

4. The microneedle array chip according to claim 1, wherein the trenches are formed extending downwards from the tips of the microneedles, and the depths of the trenches are less than heights of the microneedles.

5. The microneedle array chip according to claim 1, wherein the trenches are formed downwards from the tips of the microneedle, and the depths of the trenches are equal to heights of the microneedles.

6. The microneedle array chip according to any one of claims 1-5, wherein the trenches are V-shaped trenches.

7. The microneedle array chip according to any one of claims 1-5, wherein the trenches are rectangular trenches.

8. The microneedle array chip according to any one of claims 1-5, wherein two side surfaces of each of the trenches are bevels.

9. The microneedle array chip according to any one of claims 1-5, wherein a back side of the substrate is provided with medicine storage reservoirs, and the medicine storage reservoirs are in fluid communication with the trenches through through holes.

10. The microneedle array chip according to claim 9, wherein adjacent medicine storage reservoirs are connected by a shallow groove.

11. The microneedle array chip according to any one of claims 1-5, further comprising through holes extending from bottom surfaces of the trenches to the back side of the substrate.

12. The microneedle array chip according to any one of claims 1-5, wherein the trenches are each a straight trench or formed by two straight trenches intersecting each other by any angle.

13. A microneedle array chip, comprising a substrate, **characterized in that** a front side of the substrate is provided with a plurality of microneedle clusters arranged in an array, each of the microneedle clusters is composed of at least two microneedles, a gap between the microneedles in each microneedle cluster is not less than 10 nm and not greater than 300 µm, and the gap between the microneedles in the microneedle cluster is less than an interval between the microneedle clusters on the microneedle array chip.

14. The microneedle array chip according to claim 13, wherein a back side of the substrate is provided with medicine storage reservoirs, bottom surfaces of the medicine storage reservoirs are in fluid communication with the front side of the substrate through through holes, and the through holes are located in positions between the microneedles in the microneedle clusters.

15. The microneedle array chip according to claim 14, wherein adjacent medicine storage reservoirs are connected by a shallow groove.

16. The microneedle array chip according to claim 13, further comprising through holes extending from the front side of the substrate to the back side of the substrate, and the through holes are located in positions between the microneedles in the microneedle clusters.

17. The microneedle array chip according to any one of claims 13-16, wherein each of the microneedle clusters is composed of two microneedles.

18. The microneedle array chip according to any one of claims 13-16, wherein each of the microneedle clusters is composed of more than two microneedles, and the microneedles are circumferentially arranged.

19. The microneedle array chip according to any one of claims 13-16, wherein each of the microneedle clusters is composed of four microneedles, and the four microneedles are arranged in a circumferential array or are centrosymmetrically arranged.
